# EUROPEAN PATENT SPECIFICATION

(11) **EP 3 081 197 B1**
(45) Date of publication and mention of the grant of the patent: **14.02.2018**
(21) Application number: 15197421.9
(22) Date of filing: 02.12.2015
(51) Int. Cl.: A61F 2/966, A61M 25/09

(54) **PUSHER GUIDE WIRE AND DELIVERY SYSTEM COMPRISING THE SAME**
SCHIEBERFÜHRUNGSDRAHT UND ABGABESYSTEM DAMIT
FIL DE GUIDAGE DE POUSSOIR ET SYSTÈME D'ADMINISTRATION LE COMPRENANT

(30) Priority: 15.04.2015 JP 2015083682
(43) Date of publication of application: 19.10.2016
(73) Proprietor: PENTAS Inc., Tokyo, 150-0002 (JP)
(72) Inventor: NISHIGISHI, Makoto, Nagoya-shi, Aichi 463-0024 (JP)
(74) Representative: TBK

(56) References cited:
- EP-A1- 2 777 650
- US-A1- 2006 229 697
- US-A1- 2007 198 076
- US-A1- 2010 094 394

## Description

### TECHNICAL FIELD

The present invention relates to a pusher guide wire for delivering a stent stored in a catheter to a target site, and a delivery system comprising the pusher guide wire.

### BACKGROUND ART

**Prior art which is related to this field is disclosed in document** EP 2 777 650 A1 **showing a distal capture device for a self-expanding stent, and in document** US 2007/0198076 A1 **showing a system for delivering a stent.**

Conventionally, as a method of delivering (carrying or conveying) a stent to a target site, known is a method comprising storing the stent in the distal end portion of a catheter in a state where the stent has been disposed around the outer periphery of a pusher guide wire, advancing the catheter to the target site, and then pushing the pusher guide wire in the distal end direction to release the stent to the target site through the distal end of the catheter.

For example, Patent Literature 1 discloses a system for delivering an expandable stent having a configuration comprising a core shaft; a proximal end side columnar member covering the core shaft and fixed to the core shaft; a distal end side columnar member covering the core shaft and fixed to the core shaft at a position in the distal end side relative to the proximal end side columnar member and: separated, from the proximal end side columnar member; and a middle columnar member disposed between the proximal end side columnar member and the distal end side columnar member.

### CITATION LIST

### Patent Literature

Patent Literature 1: Japanese Patent No. 4574131

### SUMMARY OF THE INVENTION

### TECHNICAL PROBLEM

However, in the case of the configuration disclosed in Patent Literature 1, when the pusher guide wire and the stent pass through the inside of a curved blood vessel, the curved pusher guide wire may disadvantageously press against the stent and the catheter, preventing the advancement and rotation of the pusher guide wire or causing the stent to bend.

Accordingly, an objective of the **useful example for understanding the invention** is to provide a pusher guide wire allowing a stent, a catheter and the like to smoothly pass through a curved blood vessel. Further, **the** objective of the present invention is to provide a delivery system capable of delivering a stent to an appropriate location.

### SOLUTION TO PROBLEM

The **useful example for understanding the invention** provides a pusher guide wire for delivering a stent to a target site, comprising a core shaft comprising a first large-diameter portion; a small-diameter portion having an outer diameter smaller than that of the first large-diameter portion at the proximal end side of the first large-diameter portion; a second large-diameter portion having an outer diameter larger than that of the small-diameter portion at the proximal end side of the small-diameter portion; and a tubular member located between a proximal end of the first large diameter portion and a distal end of the second large diameter portion wherein the small diameter portion of the core shaft is inserted through an inside of the tubular member, wherein the tubular member is not fixed to the core shaft and is capable of moving in a radial direction relative to the core shaft.

The tubular member can move in a radial direction in an appropriate manner depending on the curvature of the catheter and be brought into contact with the stent when the pusher guide wire is inserted into a curved catheter because the tubular member is not fixed to the core shaft in the aforementioned configuration. As a result, a stent can he delivered smoothly to a target site because a clearance between the catheter and the pusher guide wire can be secured. Further, the core shaft alone can be allowed to rotate easily and smoothly because the core shaft is spaced apart from the catheter (the stent) due to the presence of the tubular member. Moreover, the catheter does not rotate when the core shaft rotates, reducing a risk of damaging the inner wall of a blood vessel and the like.

Further, the **useful example for understanding the invention** provides a pusher guide wire for delivering a stent to a target site, comprising a core shaft; a first coil body covering me core shaft and fixed to the core shaft; a second coil body covering the core shaft, the second coil body that is fixed to the core shaft at a proximal end side relative to the first coil body and separated from the first coil body; and a tubular member disposed between the first coil body and the second coil body wherein the core shaft is inserted through an inside of the tubular member, wherein the tubular member is not fixed to the core shaft and is capable of moving in a radial direction relative to the core shaft.

The tubular member can move in a radial direction in an appropriate manner depending on the curvature of the catheter and be brought into contact with the stent when the pusher guide wire is inserted into a curved catheter because the tabular member is not fixed to the core shaft in the aforementioned configuration. As a result, a stent can be delivered smoothly to a target site because a clearance between the catheter and the pusher guide wire can be secured. Further, the core shaft alone can be rotated because the core shaft is spaced apart from the catheter (the stent) due to the presence of the tubular member. Moreover, the catheter does not rotate when the core shaft rotates, reducing a risk of damaging the inner wall of a blood vessel.

In the aforementioned configuration, the second coil body preferably has a tapered portion having an increasing outer diameter toward the proximal end side.

In the aforementioned configuration, a force for directing the stent in the direction toward the distal end can be exerted by bringing the tapered portion of the second coil body into contact with the stent and pressing the tapered portion of the second coil body against the stent. As described above, the stent can be delivered more smoothly to a target site without impairing the flexibility of the pusher guide wire because a separate member needs not to be provided as a means for pressing the stent.

Further, the tubular member is preferably formed of a radiopaque material.

In the aforementioned configuration, an operator can detect the position of the stent by detecting the position of the tubular member. Therefore, it is not necessary to attach a radiopaque marker member to the stent.

Moreover, the present invention provides a delivery system **as set forth in the appended claim 1. Further advantageous features are set forth in the dependent claims.** Where in the following the word invention is used and/or features are presented as optional this should be interpreted in such way that protection is sought for the invention as claimed. Methods of using the delivery system are presented as a way to understand the invention and do not form part of the invention.

In **this** configuration, the proximal end engagement portion is provided in the proximal end portion of the stent, and therefore the tapered portion of the second coil body is easily brought into contact with the stent. Consequently, a force for directing the stent in the direction toward the distal end can easily be exerted. Further, the stent can be retracted into the catheter in the middle of release by pulling the pusher guide wire back in the direction toward the proximal end and bringing the tubular member into contact with the proximal end engagement portion of the stent.

Further, the present invention provides a delivery system comprising a pusher guide wire comprising the first coil body and the second coil body and a stent to be delivered with the pusher guide wire, wherein the distal end portion of the stent has a distal end engagement portion protruding toward the core shaft, and an inner diameter of the stent at the distal engagement portion is smaller than an outer diameter of the distal end of the tubular member.

In the aforementioned configuration, a force for directing the stent in the direction toward the distal end can be exerted by bringing the tubular member into contact with the distal end engagement portion of the stent and pressing the tubular member against the distal end engagement portion of the stent. The stent can be delivered more smoothly to a target site without impairing the flexibility of the pusher guide wire because a separate member needs not to be provided as a means for pressing the stent described above. In particular, delivery is easier because a risk of bending the stent inside of the catheter is reduced as compared with a case where the stent is pushed from the proximal end portion.

### ADVANTAGEOUS EFFECT OF THE INVENTION

The pusher guide wire according to the **useful example for understanding the invention** has an advantageous
effect in that a stent, a catheter and the like can smoothly pass through the inside of a curved blood vessel. Further, the delivery system according to the present invention has an advantageous effect in that a stent can be smoothly delivered to an appropriate location.

### BRIEF DESCRIPTION OF THE DRAWINGS

Figs. 1A and 1B show a cross sectional cut view of a delivery system according to **useful example 1 for understanding the invention.**
Figs. 2A and 2B show a partial enlarged cross sectional view of the delivery system according to **useful example 1 for understanding the invention.**
Figs. 3A and 3B show a cross sectional cut view of a delivery system according to **useful example 2 for understanding the invention.**
Figs. 4A and 4B shows a partial enlarged cross sectional view of the delivery system according to **useful example 2 for understanding the invention.**
Figs. 5A and 5B show a cross sectional cut view of a delivery system according to **useful example 3 for understanding the invention.**
Figs. 6A and 6B show a partial enlarged cross sectional view of the delivery system according to **useful example 3 for understanding the invention.**
Fig. 7 shows a cross sectional cut view of a delivery system according to **embodiment 1.**
Figs. 8A and 8B show a cross sectional cut view of an operational aspect of the delivery system according to **embodiment 1.**
Fig. 9 shows a cross sectional cut view of a delivery system according to **embodiment 2.**

### DESCRIPTION OF EMBODIMENTS

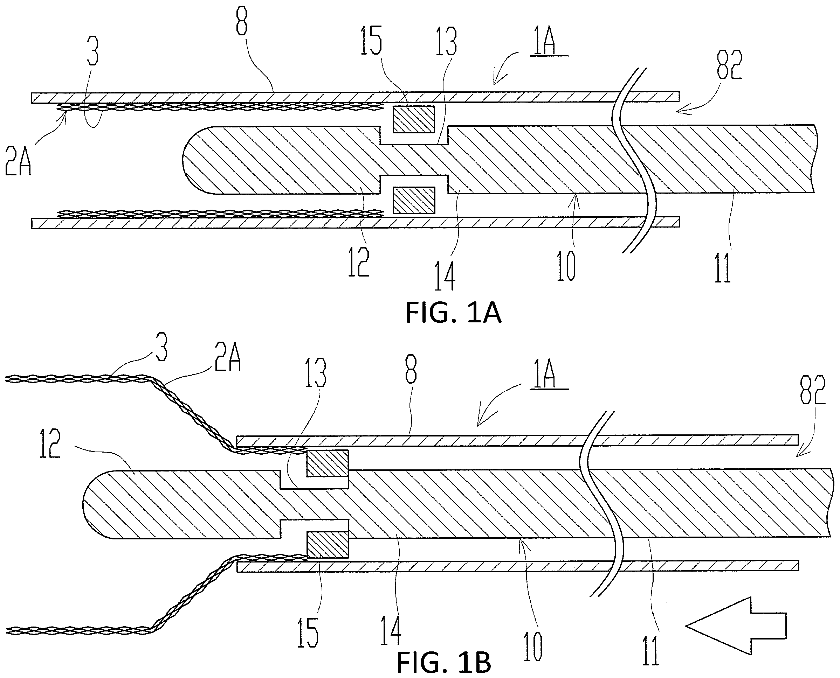

Below, **useful examples for understanding the invention** of an implementation of the pusher guide wire and the delivery system according to the present will be described in detail. Note that the present invention shall not be limited to the Examples described below, and modifications in design can be made in an appropriate manner. Further, note that the left side in Figs. 1A to 9 corresponds to the distal end side (the front side) which is to be inserted into the body, and the right side corresponds to the proximal end side (the back side) which is to be operated by an operator such as a physician.

### [Useful example 1 for understanding the present invention]

As shown in Fig. 1A, a delivery system 1A to be inserted into a catheter 8 comprises a self-expandable stent 2A and a pusher guide wire 10.

The catheter 8 is a tube-like body into which the pusher guide wire 10 can be inserted, and the stent 2A and the pusher guide wire 10 are inserted through a proximal end opening 82.

The stent 2A comprises a body portion 3 having a mesh-like cylindrical shape. Note that there is no particular limitation for a material of the stent 2A, and any known material can be used.

The pusher guide wire 10 comprises a core shaft 11 having a spherical distal end. Further, the core shaft 11 comprises a first large-diameter portion 12 having an outer diameter smaller than the inner diameter of the stent 2A, a small-diameter portion 13 having an outer diameter smaller than that of the first portion 12 at the proximal end side of the first large-diameter portion 12 and a second large-diameter portion 14 having an outer diameter larger than that of the small-diameter portion 13 at the proximal end side of the small-diameter portion 13. Note that the core shaft 11 is preferably formed of a stainless steel (SUS304, SUS316 and the like) or a superelastic alloy such as an Ni-Ti alloy.

Further, a tubular member 15 is disposed at the small-diameter portion 13 of the pusher guide wire 10 and the small-diameter portion 13 of the core shaft 11 is inserted through an inside of the tubular member 15. That is, the tubular member 15 is located between the proximal end of the first large-diameter portion 12 and the distal end of the second large-diameter portion 14.

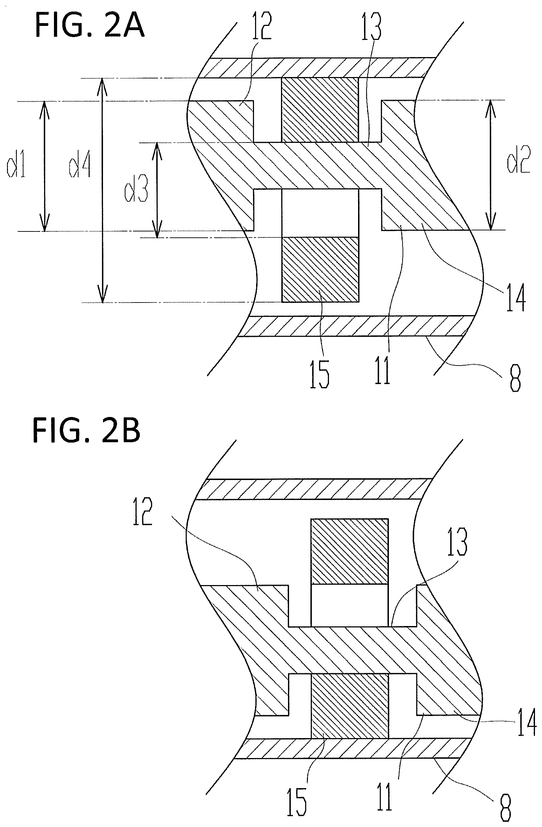

Further, the tubular member 15 is not fixed to the core shaft 11 and the dimension and shape thereof is configured so that it is capable of moving in a radial direction relative to the core shaft 11, as shown in Figs. 2A and 2B. Specifically, the inner diameter d3 of the tubular member 15 is smaller than each of the outer diameter d1 of the first large-diameter portion 12 and the outer diameter d2 of the second large-diameter portion 14 (d1 > d3, d2 > d3). Further, the outer diameter d4 of the tubular member 15 is larger than each of the outer diameter d1 of the first large-diameter portion 12 and the outer diameter d2 of the second large-diameter portion 14 (d4 > d1, d4 > d2).

Note that the tubular member 15 is preferably formed of a radiopaque material such as gold, platinum, tungsten, or an alloy comprising these elements. Thereby, an operator can detect the position of the stent 2A by detecting the position of the tubular member 15 without needing to add a radiopaque marker and the like to the stent 2A.

The tubular member 15 is not fixed to the core shaft 11 in the configuration described above, and thus the outer periphery of the tubular member 15 makes contact with the inner periphery of the catheter 8 in an appropriate manner as shown in Figs. 2A and 2B when the stent 2A and the pusher guide wire 10 pass through the inside of a curved blood vessel. Further, even in a case where the core shaft 11 is rotated, the tubular member 15 and the catheter 8 will not rotate. Therefore, a risk of damaging the inner wall of a blood vessel when rotating the core shaft 11 can be reduced.

Further, the stent 2A can be delivered smoothly to a target site because a clearance between the core shaft 11 and the catheter 8 can be secured by the presence of the tubular member 15.

Moreover, the core shaft 11 can rotate independently of the tubular member 15 and the catheter 8 as described above, leading to excellent operability.

Further, since the outer diameter of the tubular member 15 is designed to be larger than the inner diameter of the stent 2A stored inside the catheter 8, the stent 2A can be pushed out by the tubular member 15 in the direction toward the distal end within the catheter 8 as shown in Fig. 1B.

Meanwhile, examples of a method of attaching the tubular member 15 to the core shaft 11 include a configuration in which a plate-like material made of a metal or a resin is loosely wound around the small-diameter portion 13, and then both end portions of the plate-like material are joined together to form the tubular member 15. Examples further include a configuration in which the tubular member 15 is formed with a heat-shrinkable tube, and then heat is applied at a location of the small-diameter portion 13 to produce the tubular member 15 sandwiched between the first large-diameter portion 12 and the second large-diameter portion 14.

Below, the operating procedure of the delivery system 1A will be described. After advancing the catheter 8 to a target site, an operator pushes the core shaft 11 in the direction toward the distal end relative to the catheter 8 with the intention of releasing the stent 2A stored in the catheter to the target site. Then, the proximal end of the tubular member 15 makes contact with the distal end of the second large-diameter portion 14, and at the same time, the distal end of the tubular member 15 makes contact with the proximal end of the stent 2A.

Then, the stent 2A is pushed out of the catheter 8 by further pushing the core shaft 11, and the stent 2A released from the catheter 8 undergoes self-expansion as shown in Fig. 1B. Note that the pusher guide wire 10 and the catheter 8 are retrieved from the inside of the blood vessel after the stent 2A is completely pushed out of the catheter 8.

### [Useful example 2 for understanding the present invention]

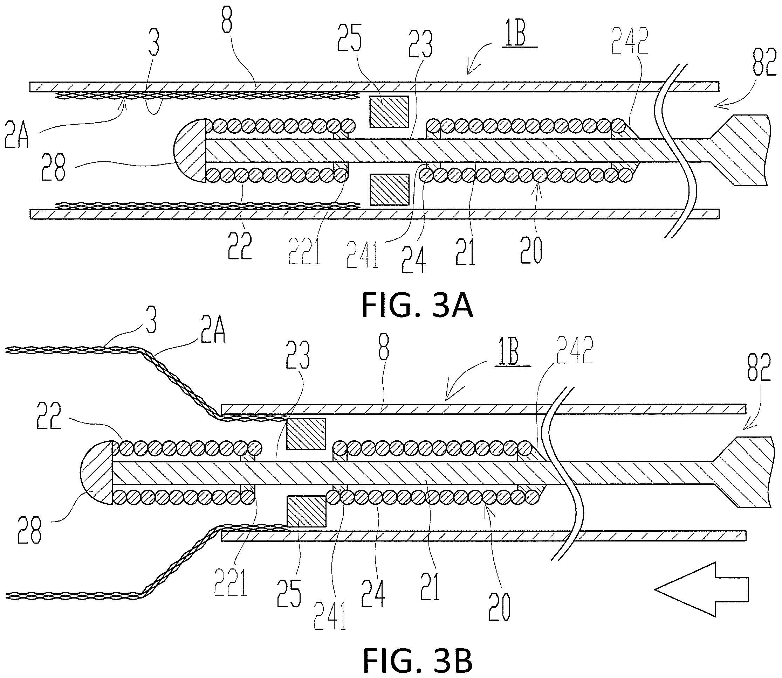

Bellow, Example 2 will be described with reference to Figs. 3A to 4B. Note that for parts common with the above Example 1, description will be omitted and the same reference numbers will be assigned in the figures.

As shown in Fig. 3A, a delivery system 1B comprises the self-expandable stent 2A and a pusher guide wire 20.

The pusher guide wire 20 comprises a first coil body 22 covering a core shaft 21 and fixed to the core shaft 21. The distal end portion of the first coil body 22 is joined to a distal end portion 28 formed at the distal end of the core shaft 21, and the proximal end portion of the first coil body 22 is bonded to the core shaft 21 through a joining region 221. Further, the pusher guide wire 20 comprises a second coil body 24 covering the core shaft 21 and fixed to the core shaft 21 at the proximal end side relative to the first coil body 22 and separated from the first coil body 22. The distal end portion of the second coil body 24 is joined to the core shaft 21 through a joining region 241, and the proximal end portion of the second coil body 24 is joined to the core shaft 21 through a joining region 242.

The first coil body 22 and the second coil body 24 are preferably formed of a stainless steel (SUS304, SUS316 and the like) or a superelastic alloy such as an Ni-Ti alloy as in the case of the core shaft 21. Further, the joining regions 221, 241, and 242 and the distal end portion 28 are preferably formed of a solder material (for example, an aluminum alloy solder, silver solder, gold solder or the like), a metal solder (for example, an Au-Sn alloy and the like) or the like.

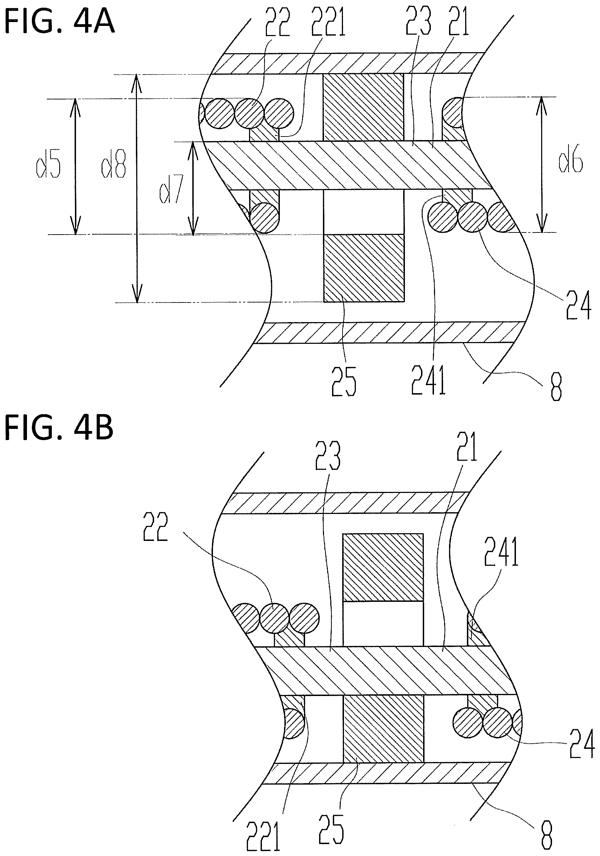

The pusher guide wire 20 further comprises a tubular member 25 which is located at a small-diameter portion 23 formed between the first coil body 22 and the second coil body 24, and the small-diameter portion 23 of the core shaft 21 is inserted through an inside of the tubular member 25. The tubular member 25 is not fixed to the core shaft 21 and is capable of moving in a radial direction relative to the core shaft 21 as shown in Figs. 4A and 4B. Specifically, the inner diameter d7 of the tubular member 25 is smaller than each of the outer diameter d5 of the first coil body 22 and the outer diameter d6 of the second coil body 24 (d5 > d7, d6 > d7). Further, the outer diameter d8 of the tubular member 25 is larger than each of the outer diameter d5 of the first coil body 22 and the outer diameter d6 of the second coil body 24 (d8 > d5, d8 > d6).

Since the tubular member 25 is not fixed to the core shaft 21 in the configuration as described above, the outer periphery of the tubular member 25 makes contact with the inner periphery of the catheter 8 in an appropriate manner when the stent 2A and the pusher guide wire 20 pass through the inside of a curved blood vessel, as shown in Figs. 4A and 4B. Further, even in a case where the core shaft 21 is rotated, the tubular member 25 and the catheter 8 will not rotate. Therefore, a risk of damaging the inner wall of a blood vessel when rotating the core shaft 21 can be reduced.

Further, the stent 2A can be delivered smoothly to a target site because a clearance between the core shaft 21 and the catheter 8 is secured by the presence of the tubular member 25.

Moreover, the core shaft 21 can rotate independently of the tubular member 25 and the catheter 8 as described above, leading to excellent operability.

Further, since the outer diameter of the tubular member 25 is larger than the inner diameter of the stent 2A stored inside the catheter 8, the stent 2A can be pushed out by the tubular member 25 in the direction toward the distal end within the catheter 8, as shown in Fig. 3B.

Below, the operating procedure of the delivery system 1B will be described. After advancing the catheter 8 to a target site, an operator pushes the core shaft 21 in the direction toward the distal end relative to the catheter 8 with the intention of releasing the stent 2A stored, in the catheter 8 to the target site. Then, the proximal end of the tubular member 25 makes contact with the distal end of the second coil body 24, and at the same time, the distal end of the tubular member 25 makes contact with the proximal end of the stent 2A.

Then, the stent 2A is pushed out of the catheter 8 by further pushing the core shaft 21, and the stent 2A released from the catheter 8 undergoes self-expansion as shown in Fig. 3B. Note that the pusher guide wire 20 and the catheter 8 are retrieved from the inside of the blood vessel after the stent 2A is completely pushed out of the catheter 8.

### [Useful example 3 for understanding the present invention]

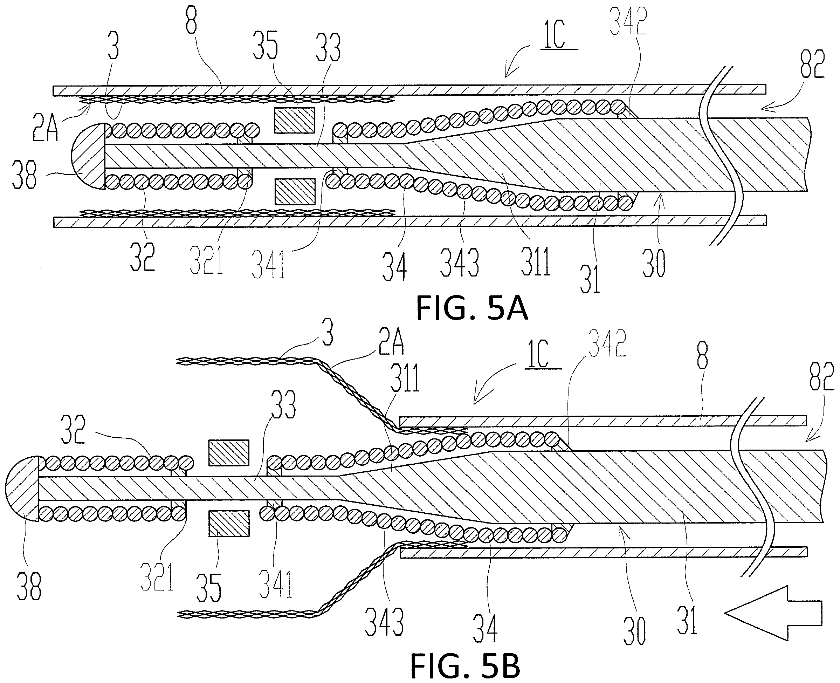

Below, Examples 3 will be described with reference to Figs. 5A to 6B. Note that for parts common with the above Examples 1 and 2, description will be omitted and the same reference numbers will be assigned in the figures.

As shown in Fig. 5A, a delivery system 1C comprises a self-expandable stent 2A and a pusher guide wire 30.

The pusher guide wire 30 comprises a first coil body 32 covering a core shaft 31 and fixed to the core shaft 31. The distal end portion of the first coil body 32 is joined to a distal end portion 38 of the core shaft 31, and the proximal end portion of the first coil body 32 is joined to the core shaft 31 through a joining region 321. Further, the pusher guide wire 30 comprises a second coil body 34 covering the core shaft 31 and fixed to the core shaft 31 at the proximal end side relative to the first coil body 32 and separated from the first coil body 32. The distal end portion of the second coil body 34 is joined to the core shaft 31 through a joining region 341, and the proximal end portion of the second coil body 34 is joined to the core shaft 31 through a joining region 342. Note that the core shaft 31 has a tapered portion 311 with an increasing outer diameter toward the proximal end in a region covered with the second coil body 34, and the second coil body 34 also has a tapered portion 343 with an increasing outer diameter toward the proximal end side.

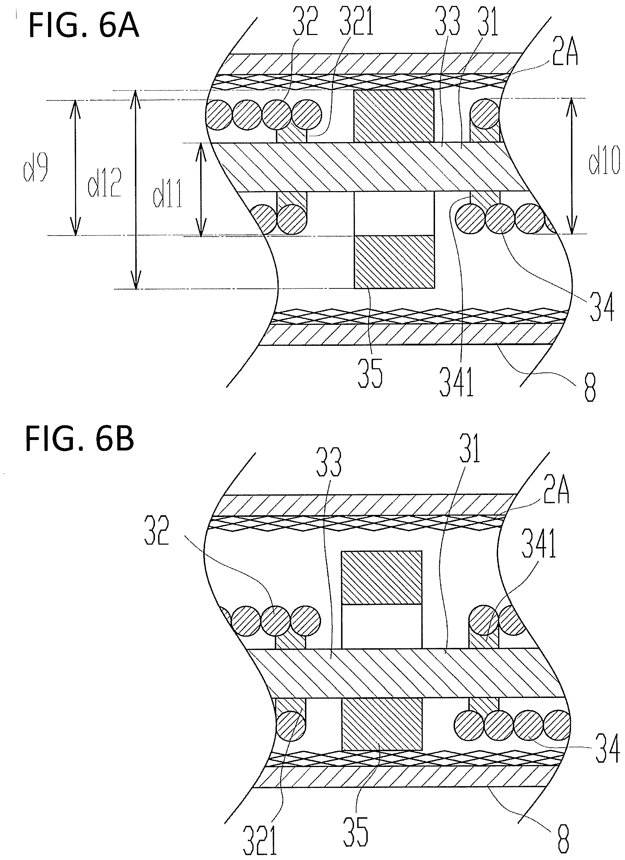

The pusher guide wire 30 further comprises a tubular member 35 is located at the small-diameter portion 33 formed between the first coil body 32 and the second coil body 34 and the small-diameter portion 33 of the core shaft 31 is inserted through an inside of the tubular member 35. The tubular member 35 is not fixed to the core shaft 31 and is capable of moving in a radial direction relative to the core shaft 31, as shown in Figs. 6A and 6B. Specifically, the inner diameter d11 of the tubular member 35 is smaller than each of the outer diameter d9 of the first coil body 32 and the outer diameter d10 of the second coil body 34 (d9 > d11, d10 > d11). Further, the outer diameter d12 of the tubular member 35 is larger than each of the outer diameter d9 of the first coil body 32 and the outer diameter d10 of the second coil body 34 (d12 > d9, d12 > d10).

Since the tubular member 35 is not fixed to the core shaft 31 in the configuration described above, the outer periphery of the tubular member 35 makes contact with the inner periphery of the stent 2A in an appropriate manner when the stent 2A and the pusher guide wire 30 pass through the inside of a curved blood vessel, as shown in Figs. 6A and 6B. Further, even in a case where the core shaft 31 is rotated, the tubular member 35, the stent 2A and the catheter 8 will not rotate. Therefore, a risk of damaging the inner wall of a blood vessel when rotating the core shaft 31 can be reduced.

Further, the stent 2A can be delivered smoothly to a target site because a clearance between the core shaft 31 and the catheter 8 can be secured by the presence of the tubular member 35.

Moreover, the core shaft 31 can rotate independently of the tubular member 35 and the catheter 8 as described above, leading to excellent operability.

Further, since the maximum outer diameter of the tapered portion 343 of the second coil body 34 is larger than the inner diameter of the stent 2A stored inside the catheter 8, the stent 2A can be pushed out by the tapered portion 343 of the second coil body 34 in the direction toward the distal end within the catheter 8, as shown in Fig. 5B. Therefore, the stent 2A can be delivered more smoothly to a target site without impairing the flexibility of the pusher guide wire 30 because a separate member needs not to be provided as a means for pressing the stent 2A.

### [Embodiment 1]

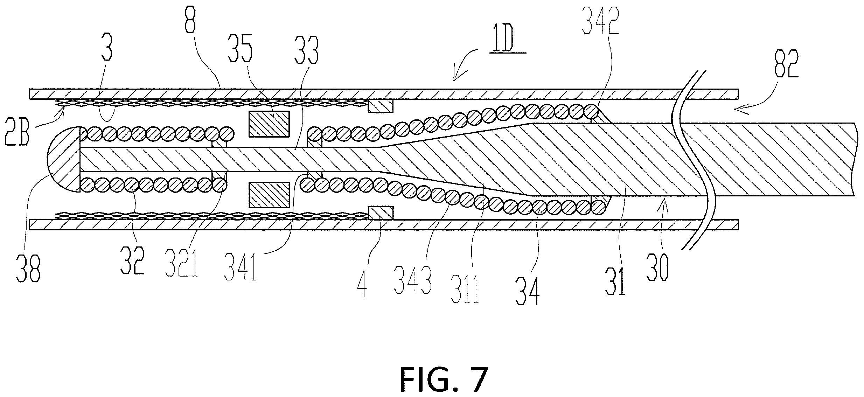

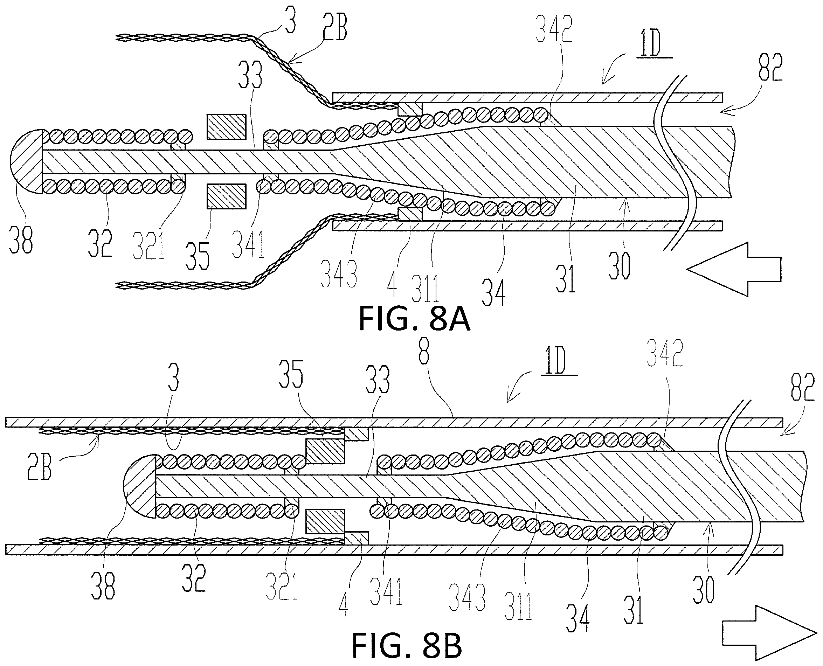

Below, **embodiment 1** will be described with reference to Figs. 7, 8A and 8B.

Note that for parts common with the above Examples 1 to 3, description will be omitted and the same reference numbers will be assigned in the figures.

As shown in Fig. 7, a delivery system 1D comprises a self-expandable stent 2B and the pusher guide wire 30.

The stent 2B comprises a body portion 3 having a mesh-like cylindrical shape. Moreover, a proximal end engagement portion 4 is formed at the proximal end portion of the body portion 3, and the proximal end engagement portion 4 protrudes toward the side of the core shaft 31. The proximal end engagement portion 4 is located between the tapered portion 343 of the second coil body 34 and the tubular member 35 in the axial direction of the core shaft 31. Note that the proximal end engagement portion 4 can be configured by joining a part of element wires of the mesh constituting the body portion 3 using, for example, a solder material. Alternatively, the proximal end engagement portion 4 can also be configured by joining, for example, a radiopaque hollow tube body or coil body to an end portion of an element wire of the mesh constituting the body portion 3.

Further, an inner diameter of the stent 2B at the proximal end engagement portion 4 stored inside the catheter 8 is designed to be smaller than the maximum outer diameter at the tapered portion 343 of the second coil body 34. Therefore, the stent 2B can be pushed out in the direction toward the distal end within the catheter 8 by pressing the proximal end engagement portion 4 with the tapered portion 343 of the second coil body 34 in the direction toward the distal end. As described above, a force for directing the stent 2B in the direction toward the distal end can easily be exerted by providing the proximal end engagement portion 4 in the stent 2B. Note that the outer diameter of the tubular member 35 is designed to be larger than the inner diameter at the proximal end engagement portion 4 of the stent 2B stored inside the catheter 8.

Below, the operating procedure of the delivery system 1D will be described. After advancing the catheter 8 to a target site, an operator pushes the core shaft 31 in the direction toward the distal end relative to the catheter 8 with the intention of releasing the stent 2B stored inside the catheter 8 to the target site. Then, as shown in Fig. 8A, the tapered portion 343 of the second coil body 34 makes contact with the proximal end engagement portion 4 of the stent 2B. Then, the stent 2B is pushed out of the catheter 8 by further pushing the core shaft 31, and the stent 2B released from the catheter 8 undergoes self-expansion.

Meanwhile, in a case where the release position of the stent 2B deviates from the target site, the pusher guide wire 30 is pulled back to the proximal end side relative to the catheter 8, and the proximal end of the first coil body 32 is then brought into contact with the distal end of the tubular member 35, and the proximal end of the tubular member 35 is also brought into contact with the proximal end engagement portion 4 of the stent 2B to further pull back the pusher guide wire 30 to the proximal end side, as shown in Fig. 8B. Thereby, the stent 2B in the middle of release is again stored inside the catheter 8. Then, the stent 2B can again be released at an appropriate position.

### [Embodiment 2]

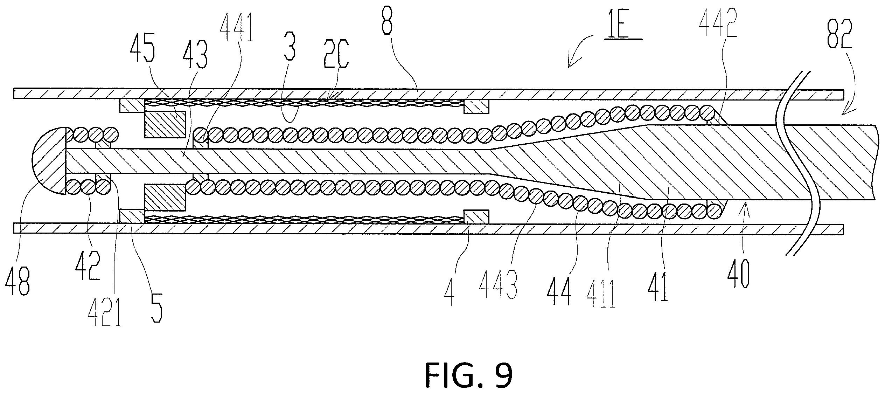

Below, **embodiment 2** will be described with reference to Fig. 9. Note that for parts common with the above Examples 1 to **3 and embodiment 1,** description will be omitted and the same reference numbers will be assigned in the figures.

As shown in Fig. 9, a delivery system 1E comprises a self-expandable stent 2C and a pusher guide wire 40.

The stent 2C has a distal end engagement portion 5 at the distal end portion of the body portion 3. The distal end engagement portion 5 protrudes toward the side of a core shaft 41.

The pusher guide wire 40 comprises a first coil body 42 covering the core shaft 41 and fixed to the core shaft 41. The distal end portion and proximal end portion of the first coil body 42 are joined to the core shaft 41 through a distal end portion 48 and a joining region 421. The pusher guide wire 40 also comprises a second coil body 44 fixed to the core shaft 41 at the proximal end side relative to the first coil body 42 and separated from the first coil body 42. The second coil body 44 is joined to the core shaft 41 through joining regions 441 and 442.

Further, the core shaft 41 also has a tapered portion 411 with an increasing diameter toward the proximal end side at a part covered with the second coil body 44. Further, the second coil body 44 also has a tapered portion 443 with an increasing outer diameter toward the proximal end.

The pusher guide wire 40 further comprises a tubular member 45 which is located at a small-diameter portion 43 formed between the first coil body 42 and the second coil body 44, and the small-diameter portion 43 of the core shaft 41 is inserted through an inside of the tubular member 45.

Here, in the case of the delivery system 1E, when the pusher guide wire 40 is pushed thereinto, the proximal end of the tubular member 45 makes contact with the distal end portion of the second coil body 44, and the distal end of the tubular member 45 makes contact with the distal end engagement portion 5 of the stent 2C. At this time, the proximal end engagement portion 4 of the stent 2C does not make contact with the pusher guide wire 40.

In the aforementioned configuration, the distal end engagement portion 5 of the stent 2C can be pushed by pushing the pusher guide wire 40 to exert a force for directing the stent 2C in the direction toward the distal end. In this case, a risk of bending the stent 2C inside the catheter 8 can be reduced, allowing the stent 2C to be delivered more smoothly.

The dimension and shape of each part described in the aforementioned Examples **1** to **3 and embodiments 1 and 2** may be freely selected in an appropriate manner within a range that does not depart from the spirit of the present invention. Further, materials used shall not be limited to those described in the aforementioned Examples 1 to **3 and embodiments 1 and 2.** For example, in the aforementioned Examples **2, 3 and embodiments 1 and 2, the first coil bodies 22,** 32, 42 and the second coil bodies 24, 34, 44 are formed by winding one wire. Without being limited to such a construction, the first coil bodies 22, 32, 42 and the second coil bodies 24, 34, 44 may be formed by spirally twisting together two or more wires or by spirally twisting together two or more twisted wires formed by twisting together two or more wires. As described above, the rotational operability of the pusher guide wires 20, 30, 40 can be improved in a case where the first coil bodies 22, 32, 42 and the second coil bodies 24, 34, 44 are formed by twisting together two or more wires or two or more twisted wires. In particular, in a case where the tapered portion 343 of the second coil body 34 is brought into contact with the proximal end engagement portion 4 of the stent 2B to push the stent 2B in the direction toward the distal end as shown in **embodiment 1,** or in a case where the proximal end of the tubular member 45 is brought into contact with the distal end portion of the second coil body 44, and the distal end of the tubular member 45 is brought into contact with the distal end engagement portion 5 of the stent 2C to push the stent 2C in the direction toward the distal end as shown in **embodiment 2,** the second coil bodies 34, 44 are preferably formed by spirally twisting together two or more wires or by spirally twisting together two or more twisted wires. In the pusher guide wires 30, 40 comprising the second coil bodies 34 and 44 as described above, rotational operation can be allowed while pushing in the direction toward the distal end, and thus the stents 2B and 2C can be delivered more smoothly even to the inside of a curved blood vessel.

### Description of Symbols

1A to 1E delivery system
2A to 2C stent
4 proximal end engagement portion
5 distal end engagement portion
10, 20, 30, 40 pusher guide wire
11, 21, 31, 41 core shaft
12 first large-diameter portion
13 small-diameter portion
14 second large-diameter portion
15, 25, 35, 45 tubular member
22,32,42 first coil body
24, 34, 44 second coil body
343,443 tapered portion

## Claims

1. A delivery system (1D, 1E) comprising:
a catheter (8),
a pusher guide wire (30, 40) inserted into the catheter (8) for delivering a stent (2B, 2C) to a target site, comprising:
a core shaft (31, 41);
a first coil body (32, 42) covering the core shaft (31, 41) and fixed to the core shaft (31, 41);
a second coil body (34, 44) covering the core shaft (31, 41), the second coil body (34, 44) that is fixed to the core shaft (31, 41) at a proximal end side relative to the first coil body (32, 42) and separated from the first coil body (32, 42); and,
a tubular member (35, 45) disposed between the first coil body (32, 42) and the second coil body (34, 44) wherein the core shaft (31, 41) is inserted through an inside of the tubular member (35, 45); and
the stent (2B, 2C) to be delivered by the pusher guide wire (30, 40),
**characterized in that**
the tubular member (35, 45) is not fixed to the core shaft (21, 31, 41), and is capable of moving in a radial direction relative to the core shaft (31, 41) such that the outer periphery of the tubular member (35, 45) makes contact with the inner periphery of the stent (2B, 2C) depending on a curvature of the catheter (8) when the stent (2B, 2C) and the pusher guide wire (30, 40) pass through the inside of a curved blood vessel,
wherein the second coil body (34, 44) has a tapered portion (343, 443) with an increasing outer diameter toward a proximal end side,
wherein a proximal end portion of the stent (2B, 2C) has a proximal end engagement portion (4) protruding toward the core shaft (31, 41),
the proximal end engagement portion (4) of the stent (2B, 2C) is located between the tapered portion (343, 443) and the tubular member (35, 45) in an axial direction of the core shaft (31, 41), and
an inner diameter of the stent (2B, 2C) at the proximal end engagement portion (4) is smaller than an outer diameter of the second coil body (34, 44) at the tapered portion (343, 443).

2. The delivery system (1D, 1E) according to claim 1,
wherein the tubular member (35, 45) is formed of a radiopaque material.

3. The delivery system (1E) according to claim 1 or 2,
wherein a distal end portion of the stent (2C) has a distal end engagement portion (5) protruding toward the core shaft (41), and
an inner diameter of the stent (2C) at the distal end engagement portion (5) is smaller than an outer diameter of a distal end of the tubular member (45).

4. The delivery system (1E) according to claim 3,
wherein when the stent (2C) is delivered by the pusher guide wire (40), the proximal end of the tubular member (45) is configured to make contact with the distal end portion of the second coil body (44), the distal end of the tubular member (45) is configured to make contact with the distal end engagement portion (5) of the stent (2C), and the proximal end engagement portion (4) of the stent (2C) is configured not to make contact with the pusher guide wire (40).

## Patentansprüche

1. Zuführsystem (1D, 1E), mit:
einem Katheter (8),
einem Schieberführungsdraht (30, 40) zum Zuführen eines Stents (2B, 2C) an einen Zielort, der in dem Katheter (8) eingeführt ist, mit:
einer Kernwelle (31, 41);
einem ersten Spulenkörper (32, 42), der die Kernwelle (31, 41) bedeckt und an der Kernwelle (31, 41) befestigt ist;
einem zweiten Spulenkörper (34, 44), der die Kernwelle (31, 41) bedeckt, wobei der zweite Spulenkörper (34, 44) an einer dem ersten Spulenkörper (32, 42) nahen Endseite der Kernwelle (31, 41) und von dem ersten Spulenkörper (32, 42) getrennt befestigt ist; und
einem Schlauchelement (35, 45), das zwischen dem ersten Spulenkörper (32, 42) und dem zweiten Spulenkörper (34, 44) angeordnet ist, wobei die Kernwelle (31, 41) durch einen Innenraum des Schlauchelementes (35, 45) eingeführt ist; und
dem Stent (2B, 2C), der durch den Schieberführungsdraht (30, 40) zuzuführen ist,
**dadurch gekennzeichnet, dass**
das Schlauchelement (35, 45) nicht an der Kernwelle (21, 31, 41) befestigt ist, und relativ zur Kernwelle (31, 41) in einer radialen Richtung derart beweglich ist, dass die Außenseite des Schlauchelementes (35, 45) mit der Innenseite des Stents (2B, 2C) in Abhängigkeit von einer Biegung des Katheters (8) in Kontakt gelangt, wenn der Stent (2B, 2C) und der Schieberführungsdraht (30, 40) durch das Innere eines gebogenen Blutgefäßes hindurchpassieren,
wobei der zweite Spulenkörper (34, 44) einen sich verjüngenden Abschnitt (343, 443) mit einem äußeren Durchmesser aufweist, dessen Außendurchmesser zu einer nahen Endseite hin zunimmt,
wobei ein naher Endabschnitt des Stents (2B, 2C) einen Eingriffsabschnitt an dem nahen Ende (4) aufweist, der zu der Kernwelle (31, 41) hin hervorsteht,
der Eingriffsabschnitt an dem nahen Ende (4) des Stents (2B, 2C) in einer axialen Richtung der Kernwelle (31, 41) zwischen dem sich verjüngenden Abschnitt (343, 443) und dem Schlauchelement (35, 45) angeordnet ist, und
ein Innendurchmesser des Stents (2B, 2C) an dem Eingriffsabschnitt an dem nahen Ende (4) kleiner als ein Außendurchmesser des zweiten Spulenkörpers (34, 44) an dem sich verjüngenden Abschnitt (343, 443) ist.

2. Zuführsystem (1D, 1E) nach Anspruch 1, wobei
das Schlauchelement (35, 45) aus einem röntgendichten Material ausgebildet ist.

3. Zuführsystem (1E) nach Anspruch 1 oder 2, wobei
ein Abschnitt am fernen Ende des Stents (2C) einen Eingriffsabschnitt des fernen Endes (5) aufweist, der zu der Kernwelle (41) hin hervorsteht, und
ein Innendurchmesser des Stents (2C) an dem Eingriffsabschnitt des fernen Endes (5) kleiner als ein Außendurchmesser eines fernen Endes des Schlauchelementes (45) ist.

4. Zuführsystem (1E) nach Anspruch 3, wobei
wenn der Stent (2C) durch den Schieberführungsdraht (40) zugeführt wird, dann ist das nahe Ende des Schlauchelementes (45) dazu eingerichtet, in Kontakt mit dem Abschnitt des fernen Endes des zweiten Spulenkörpers (44) zu gelangen, das ferne Ende des Schlauchelementes (45) ist dazu eingerichtet, in Kontakt mit dem Eingriffsabschnitt des fernen Endes (5) des Stents (2C) zu gelangen, und der Eingriffsabschnitt des nahen Endes (4) des Stents (2C) ist dazu eingerichtet, nicht in Kontakt mit dem Schieberführungsdraht (40) zu gelangen.

## Revendications

1. Système de pose (1D, 1E) comprenant :
un cathéter (8),
un fil-guide poussoir (30, 40) inséré dans le cathéter (8) pour poser un stent (2B, 2C) sur un site cible, comprenant :
un arbre central (31, 41) ;
un premier corps hélicoïdal (32, 42) recouvrant l'arbre central (31, 41) et fixé à l'arbre central (31, 41) ;
un second corps hélicoïdal (34, 44) recouvrant l'arbre central (31, 41), le second corps hélicoïdal (34, 44) qui est fixé à l'arbre central (31, 41) au niveau d'un côté d'extrémité proximale par rapport au premier corps hélicoïdal (32, 42) et séparé du premier corps hélicoïdal (32, 42) ; et
un élément tubulaire (35, 45) disposé entre le premier corps hélicoïdal (32, 42) et le second corps hélicoïdal (34, 44), dans lequel l'arbre central (31, 41) est inséré à travers un intérieur de l'élément tubulaire (35, 45) ; et
le stent (2B, 2C) à poser grâce au fil-guide poussoir (30, 40),
**caractérisé en ce que** :
l'élément tubulaire (35, 45) n'est pas pas fixé à l'arbre central (21, 31, 41) et peut se dépasser dans une direction radiale par rapport à l'arbre central (31, 41) de sorte que la périphérie externe de l'élément tubulaire (35, 45) établit le contact avec la périphérie externe du stent (2B, 2C) en fonction d'une courbure du cathéter (8) lorsque le stent (2B, 2C) et le fil-guide poussoir (30, 40) passent à l'intérieur d'un vaisseau sanguin incurvé,
dans lequel le second corps hélicoïdal (34, 44) a une partie progressivement rétrécie (343, 443) avec un diamètre externe croissant vers un côté d'extrémité proximale,
dans lequel une partie d'extrémité proximale du stent (2B, 2C) a une partie de mise en prise d'extrémité proximale (4) faisant saillie vers l'arbre central (31, 41),
la partie de mise en prise d'extrémité proximale (4) du stent (2B, 2C) est positionnée entre la partie progressivement rétrécie (343, 443) et l'élément tubulaire (35, 45) dans une direction axiale de l'arbre central (31, 41), et
un diamètre interne du stent (2B, 2C) au niveau d'une partie de mise en prise d'extrémité proximale (4) est inférieur à un diamètre externe du second corps hélicoïdal (34, 44) au niveau de la partie progressivement rétrécie (343, 443).

2. Système de pose (1D, 1E) selon la revendication 1, dans lequel l'élément tubulaire (35, 45) est formé avec un matériau radio-opaque.

3. Système de pose (1E) selon la revendication 1 ou 2, dans lequel une partie d'extrémité distale du stent (2C) a une partie de mise en prise d'extrémité distale (5) faisant saillie vers l'arbre central (41), et
un diamètre interne du stent (2C) au niveau de la partie de mise en prise d'extrémité distale (5) est inférieur à un diamètre externe d'une extrémité distale de l'élément tubulaire (45).

4. Système de pose (1E) selon la revendication 3,
dans lequel lorsque le stent (2C) est posé grâce au fil-guide poussoir (40), l'extrémité proximale de l'élément tubulaire (45) est configurée pour établir le contact avec la partie d'extrémité distale du second corps hélicoïdal (44), l'extrémité distale de l'élément tubulaire (45) est configurée pour établir le contact avec la partie de mise en prise d'extrémité distale (5) du stent (2C), et la partie de mise en prise d'extrémité proximale (4) du stent (2C) est configurée pour ne pas établir le contact avec le fil-guide poussoir (40).
